(19) Europäisches Patentamt
European Patent Office
Office européen des brevets

(11) EP 0 659 405 B1

(12) FASCICULE DE BREVET EUROPEEN

(45) Date de publication et mention de la délivrance du brevet:
**25.02.1998 Bulletin 1998/09**

(51) Int. Cl.⁶: **A61K 7/48**, A61K 7/06

(21) Numéro de dépôt: **94402512.1**

(22) Date de dépôt: **07.11.1994**

# (54) Composition cosmétique contenant un mélange de polymères conditionneurs

Kosmetische Zusammensetzung enthaltend eine Mischung aus Konditionnierungspolymere

Cosmetic composition containing a blend of conditionning polymers

(84) Etats contractants désignés:
**DE ES FR GB IT**

(30) Priorité: **27.12.1993 FR 9315691**

(43) Date de publication de la demande:
**28.06.1995 Bulletin 1995/26**

(73) Titulaire: **L'OREAL**
**75008 Paris (FR)**

(72) Inventeurs:
- **Cauwet, Danièle**
  **F-75011 Paris (FR)**
- **Dubief, Claude**
  **F-78150 Le Chesnay (FR)**

(74) Mandataire:
**Tezier Herman, Béatrice**
**L'OREAL,**
**Département Propriété Industrielle,**
**90, rue du Gal Roguet**
**92583 Clichy Cédex (FR)**

(56) Documents cités:
**EP-A- 0 557 203**
**GB-A- 2 063 671**
**GB-A- 2 114 580**

EP 0 659 405 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Printed by Xerox (UK) Business Services
2.15.11/3.4

## Description

L'invention a pour objet des compositions cosmétiques pour les cheveux et la peau contenant des polymères conditionneurs.

On a déja préconisé dans des compositions pour le lavage ou le soin des cheveux l'utilisation de polymères conditionneurs, notamment cationiques, pour faciliter le démêlage des cheveux et pour leur communiquer douceur et souplesse. Cependant, l'usage des polymères cationiques dans ce but présente divers inconvénients. En raison de leur forte affinité pour les cheveux, certains de ces polymères se déposent de façon importante lors d'utilisations répétées, et conduisent à des effets indésirables tel qu'un toucher désagréable, un raidissement des cheveux, et une adhésion interfibre affectant le coiffage. Ces inconvénients sont accentués dans le cas de cheveux fins, qui manquent de tenue, de nervosité et de volume.

Parmi les documents de l'art antérieur décrivant l'utilisation de polymères cationiques comme agents cosmétiques, on peut citer la demande de brevet FR-A-2 270 846 qui décrit l'utilisation de polymères quaternisés.

L'utilisation de ces polymères quaternisés comme seuls agents de traitement cosmétique n'est pas complètement satisfaisante quant à la tenue des cheveux.

On a également préconisé pour améliorer les propriétés conditionnantes des produits capillaires, l'utilisation de polymères amphotères tels que ceux décrits dans la demande de brevet EP-A-269 243. Cependant, les compositions contenant uniquement ces polymères ne permettent pas d'obtenir une douceur et un démêlage suffisants.

Par ailleurs, dans les demandes FR-A-2 470 596 et FR-A-2 519 863, il est prévu des compositions cosmétiques pour le traitement des cheveux contenant l'association d'un polymère cationique et d'un polymère amphotère. Si ces compositions sont supérieures aux compositions ne contenant qu'un polymère cationique ou qu'un polymère amphotère, elles ne donnent cependant pas entière satisfaction en ce qui concerne les propriétés de démêlage et de douceur conférées aux cheveux.

La demanderesse a maintenant découvert que l'association de certains polymères conditionneurs décrits dans les documents antérieurs qui viennent d'être mentionnés, lorsqu'ils sont utilisés dans un rapport donné permet de remédier à ces inconvénients du fait d'un effet de synergie.

Cette association apporte des propriétés cosmétiques nettement améliorées par rapport aux propriétés obtenues avec l'un ou l'autre des constituants utilisé seul, ainsi que par rapport aux associations des deux constituants utilisés dans des rapports sortant du domaine de l'invention.

On a découvert en particulier que les compositions obtenues selon l'invention apportent une amélioration du démêlage (notamment sur cheveux humides) ainsi qu'une amélioration de la douceur des cheveux. En outre, les cheveux ne sont pas alourdis après des applications répétées.

Par ailleurs, les compositions de l'invention appliquées sur la peau notamment sous forme de bain moussant ou de gel douche, apportent une amélioration de la douceur de la peau.

L'invention a donc pour objet des compositions cosmétiques caractérisées par le fait qu'elles contiennent :

- au moins un polymère (a) de polyammonium quaternaire constitué de motifs récurrents répondant à la formule :

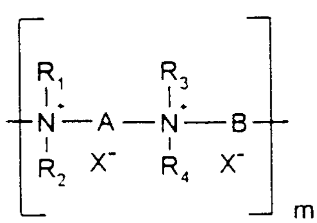

(a)

dans laquelle :

- A et B, identiques ou différents, peuvent représenter des groupements polyméthyléniques contenant de 2 à 20 atomes de carbone pouvant être linéaires ou ramifiés, saturés ou insaturés et pouvant contenir, intercalés dans la chaîne principale un ou plusieurs groupements $-CH_2-Y-CH_2-$ avec Y désignant 0, S, SO, $SO_2$ ou -CHOH-
- $X^-$ est un anion dérivé d'un acide minéral ou organique.
- m est tel que la masse moléculaire est comprise entre 1 000 et 100 000 déterminée par chromatographie par perméation de gel.
- $R_1$, $R_2$, $R_3$ et $R_4$, identiques ou différents désignent un radical alkyle ou hydroxyalkyle ayant de 1 à 4 atomes de carbone environ.

et

- au moins un polymère (b) constitué de 70 à 90 % en poids environ d'unités diallyldialkylammonium dans lesquelles

le radical alkyle contient de 1 à 18 atomes de carbone et de 30 % à 10 % en poids environ d'unités acrylique ou méthacrylique, dans un rapport pondéral $\frac{(a)}{(b)}$ strictement inférieur à 1.

Parmi les polymères (a), on préfère ceux qui sont constitués de motifs récurrents répondant à la formule :

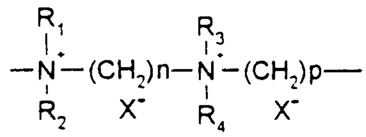 (a)

dans laquelle $R_1$, $R_2$, $R_3$ et $R_4$, identiques ou différents, désignent un radical alkyle ou hydroxyalkyle ayant de 1 à 4 atomes de carbone environ, n et p sont des nombres entiers variant de 2 à 20 environ et, $X^-$ est un anion dérivé d'un acide minéral ou organique.

Encore plus particulièrement, on préfère les composés dans lesquels $R_1$, $R_2$, $R_3$ et $R_4$ représentent un radical méthyle ou éthyle et $X^-$ est un atome d'halogène tel que un atome de chlore, d'iode ou de brome.

Un composé de formule (a) particulièrement préféré est celui pour lequel $R_1$, $R_2$, $R_3$ et $R_4$, représentent un radical méthyle et n = 3, p = 6 et X = Cl.

Un autre composé de formule (a) particulièrement préféré est celui pour lequel $R_1$et $R_2$, représentent un radical méthyle, $R_3$ et $R_4$ représentent un radical éthyle et n = p = 3 et X = Br.

Parmi les polymères (b), on préfère les copolymères de chlorure de diallyldiméthylammonium ou de diallyldiéthylammonium et d'acide acrylique d'un poids moléculaire déterminé par chromatographie par perméation de gel, compris entre 50.000 et 10.000.000 et de préférence entre 200.000 et 5.000.000.

Un polymère de ce type particulièrement préféré est le copolymère de chlorure de diallyldiméthylammonium et d'acide acrylique (80/20 en poids) vendu en solution à 35 % de matière active par la société CALGON CORP sous la dénomination MERQUAT 280®.

De préférence, le rapport $\frac{(a)}{(b)}$ est inférieur ou égal à 0,75. Encore plus préférentiellement, il est inférieur ou égal à 0,5.

Dans les compositions selon l'invention, la proportion pondérale du polymère (a) est préférentiellement comprise entre 0,05 % et 4 % et, encore plus préférentiellement entre 0,1 % et 3 % ; celle du polymère (b) peut varier de préférence entre 0,1 % et 8 % et encore plus préférentiellement entre 0,2 % et 6 % par rapport au poids total de la composition.

Les compositions de l'invention contiennent avantageusement en outre au moins un agent tensioactif en une quantité comprise entre 0,1 % et 40 % en poids environ, de préférence entre 3 % et 40 % et encore plus préférentiellement entre 5 et 30 %.

Cet agent tensioactif peut être choisi parmi les agents tensioactifs anioniques, amphotères, zwitterioniques, non-ioniques, cationiques ou leurs mélanges.

Parmi les tensioactifs anioniques, on peut citer les sels (en particulier sels alcalins, notamment de sodium, sels d'ammonium, sels d'amines, sels d'aminoalcools ou sels de magnésium) des composés suivants : les alkyléthersulfates, alkylamidoéthersulfates, alkylarylpolyéthersulfates, monoglycérides sulfates ; les alkylsulfonates, alkylsulfates, alkylphosphates, alkylamidesulfonates, alkylarylsulfonates, $\alpha$-oléfine-sulfonates, paraffine-sulfonates ; les alkylsulfosuccinates, les alkyléthersulfosuccinates, les alkylamide sulfosuccinates ; les alkylsulfosuccinamates ; les alkylsulfoacétates ; les alkylètherphosphates ; les acylsarcosinates et les N-acyltaurates. Le radical alkyle ou acyle de ces différents composés comporte de préférence de 12 à 20 atomes de carbone.

Parmi les tensioactifs anioniques, on peut encore citer les sels d'acides gras tels que les sels des acides oléique, ricinoléique, palmitique, stéarique, les acides d'huile de coprah ou d'huile de coprah hydrogénée : les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides éthers carboxyliques polyoxyalkylénés, en particulier ceux comportant de 2 à 24 groupements oxyde d'éthylène, et leurs mélanges. Les tensioactifs anioniques du type acide éther carboxylique polyoxyalkyléné sont en particulier ceux qui répondent à la formule (i) suivante :

$$R_5\text{-}(OC_3H_6)q\text{—}(OC_2H_4)r\text{—}OCH_2COOA \qquad \text{(i)}$$

dans laquelle :

$R_5$ désigne un groupement alkyle ou alkényle linéaire ou ramifié en $C_8$-$C_{22}$, un alkyl ($C_8$-$C_9$)-phényle, un groupe-

ment R'-CONH-$CH_2$- avec R' désignant un alkyle ou alkényle en $C_{11}$-$C_{21}$,
q est un nombre entier ou décimal pouvant varier de 0 à 6, et
r est un nombre entier ou décimal pouvant varier de 2 à 24 et de préférence de 3 à 10.

A désigne H, ammonium, Na, K, Li, Mg ou un reste monoéthanolamine ou triéthanolamine. On peut également utiliser des mélanges de composés de formule (i) en particulier des mélanges dans lesquels les groupements $R_5$ sont différents.

Des composés de formule (i) sont vendus par exemple par la Société CHEM Y sous les dénominations AKYPOS® (NP40, NP70, OP40, OP80, RLM25, RLM38, RLMQ 38 NV, RLM 45, RLM 45 NV, RLM 100, RLM 100 NV, RO 20, RO 90, RCS 60, RS 60, RS 100, RO 50) ou par la Société SANDOZ sous les dénominations SANDOPAN® (DTC Acid, DTC).

Selon un mode de réalisation préféré de l'invention, on utilise comme agent tensioactif anionique au moins un composé du type acide carboxylique de formule (i) indiquée ci-dessus, dans laquelle $R_4$ désigne un radical alkyle ($C_{12}$-$C_{14}$), oléyle, cétyle ou stéaryle, A désigne un atome d'hydrogène ou de sodium, q = 0 et r est compris entre 3 et 10. On utilise par exemple le produit commercial vendu par la Société CHEM Y sous la dénomination RLM 45® ($R_5$ : alkyle ($C_{12}$-$C_{14}$), valeur moyenne de r = 4,5, q = 0 et A = H).

Les agents tensioactifs non-ioniques peuvent être choisis parmi les alcools, les alphadiols, les alkylphénols ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 les amines grasses polyéthoxylées ayant de préférence 2 à 30 moles d'oxyde d'éthylène : les esters d'acides gras du sorbitan oxyéthylénés ayant de 2 à 30 moles d'oxyde d'éthylène les esters d'acides gras du sucrose, les esters d'acides gras du polyéthylèneglycol, les alkylpolyglycosides, les dérivés de N-alkyl glucamine, les oxydes d'amines tels que les oxydes d'alkyl ($C_{10}$ - $C_{14}$) amines ou les oxydes de N-acylaminopropylmorpholine. Les alkylpolyglycosides et les polyglycérolés font partie des tensioactifs non-ioniques plus particulièrement préférés.

Les agents tensioactifs amphotères ou zwitterioniques sont notamment des dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) on peut citer encore les alkyl ($C_8$-$C_{20}$) bétaïnes, les sulfobétaïnes, les alkyl ($C_8$-$C_{20}$) amidoalkyl ($C_1$-$C_6$) betaïnes ou les alkyl ($C_8$-$C_{20}$) amidoalkyl ($C_1$-$C_6$) sulfobétaïnes.

Parmi les dérivés d'amines, on peut citer les produits vendus sous la dénomination MIRANOL, tels que décrits dans les brevets US-2 528 378 et US-2 781 354 et classés dans le dictionnaire CTFA, 3ème édition, 1982, sous les dénominations Amphocarboxyglycinates et Amphocarboxypropionates de structures respectives :

$$R_6\text{-CONHCH}_2\text{CH}_2\text{-N}(R_7)(R_8)(\text{CH}_2\text{COO}^-)$$

dans laquelle : $R_6$ désigne un radical alkyle d'un acide $R_6$-COOH présent dans l'huile de coprah hydrolysée, un radical heptyle, nonyle ou undécyle, $R_7$ désigne un groupement bêta-hydroxyéthyle et $R_8$ un groupement carboxyméthyle ; et

$$R_9\text{-CONHCH}_2\text{CH}_2\text{-N(B)(C)}$$

dans laquelle :

B représente -$CH_2CH_2OX'$, C représente -$(CH_2)_z$-Y', avec z = 1 ou 2,
X' désigne le groupement -$CH_2CH_2$-COOH ou un atome d'hydrogène
Y' désigne -COOH ou le radical -$CH_2$ - CHOH - $SO_3H$
$R_9$ désigne un radical alkyle d'un acide $R_9$ -COOH présent dans l'huile de coprah ou dans l'huile de lin hydrolysée, un radical alkyle, notamment en $C_7$, $C_9$, $C_{11}$ ou $C_{13}$, un radical alkyle en $C_{17}$ et sa forme iso, un radical $C_{17}$ insaturé.

A titre d'exemple on peut citer le cocoamphocarboxyglycinate vendu sous la dénomination commerciale MIRANOL C2M® concentré par la Société MIRANOL.

Parmi les tensioactifs cationiques on peut citer en particulier : les sels d'amines grasses primaires, secondaires ou tertiaires, éventuellement polyoxyalkylénées les sels d'ammonium quaternaire tels que les chlorures ou les bromures

de tétraalkylammonium, d'alkylamidoalkyltrialkylammonium, de trialkylbenzylammonium, de trialkylhydroxyalkylammonium ou d'alkylpyridinium; les dérivés d'imidazoline ; ou les oxydes d'amines à caractère cationique.

La concentration de ces agents tensio-actifs cationiques, est de préférence comprise entre 0,1 % et 10 % en poids par rapport au poids total de la composition.

Les tensioactifs anioniques sont de préférence utilisés en mélange avec des tensioactifs amphotères. Dans ce cas, le rapport pondéral des premiers aux seconds peut varier de 0,5 à 10 et de préférence de 1 à 5.

Les compositions conformes à l'invention peuvent contenir en outre des adjuvants usuels.

Ce sont par exemple des parfums, des solvants, des agents conservateurs, séquestrants, épaississants, adoucissants, modificateurs de mousse, acidifiants ou alcalinisants.

Les épaississants peuvent être choisis notamment parmi l'alginate de sodium, la gomme arabique, les dérivés cellulosiques tels que la méthylcellulose, l'hydroxyméthylcellulose, l'hydroxyéthylcellulose, l'hydroxypropylcellulose, l'hydroxypropylméthylcellulose, la gomme de guar ou ses dérivés, les gommes de xanthane, les scléroglucanes , les acides polyacryliques réticulés, l'oléate de propylèneglycol oxyéthyléné à 55 moles d'oxyde d'éthylène et des éthers d'alcools gras ayant de 27 à 44 atomes de carbone.

L'épaississant peut également être obtenu par mélange du polyéthylèneglycol et de stéarates ou de distéarates de polyéthylèneglycol ou par mélange d'esters phosphoriques et d'amides.

Ces épaississants sont utilisés de préférence dans des proportions pouvant aller de 0,5 à 5 % en poids par rapport au poids total de la composition.

Le milieu aqueux peut contenir, outre de l'eau, des solvants cosmétiquement acceptables tels que des monoalcools, des polyalcools, des éthers de glycol ou des esters d'acides gras, utilisés seuls ou en mélange. Parmi ces solvants, on peut plus particulièrement mentionner les alcools inférieurs tels que l'éthanol, l'isopropanol, les polyalcools tels que l'éthylèneglycol, le diéthylèneglycol, le propylèneglycol, les éthers de glycol et les alkyl éthers de glycol ou de diéthylèneglycol.

Les solvants sont utilisés de préférence dans des proportions comprises entre 0,5 et 10 % en poids par rapport au poids total de la composition.

Les compositions selon l'invention peuvent également contenir des colorants, des agents modificateurs de viscosité, des agents nacrants, des agents hydratants, anti-pelliculaires, anti-séborrhéiques, des filtres solaires, des silicones volatiles ou non, organomodifiées ou non, d'autres agents de conditionnement que ceux de l'invention tels que des composés cationiques polymériques ou non, des huiles hydrocarbonées, des protéines, des vitamines, etc.

Le pH des compositions selon l'invention est généralement compris entre 4 et 8 et de préférence entre 5 et 7.

Les compositions conformes à l'invention peuvent être utilisées pour le lavage et le traitement des cheveux et/ou de la peau.

Les compositions de l'invention peuvent plus particulièrement se présenter sous forme d'après-shampooing à rincer ou non, de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.

Les compositions de l'invention peuvent encore se présenter sous la forme de compositions lavantes pour le corps, et en particulier sous la forme de solutions ou de gels pour le bain ou la douche ou de produits démaquillants.

Les compositions selon l'invention peuvent également se présenter sous forme de lotions aqueuses ou hydroalcooliques pour le soin de la peau et des cheveux.

L'homme de l'art détermine parmi les divers additifs énumérés ci-avant ceux convenant à l'application recherchée.

Les exemples suivants illustrent l'invention.

EXEMPLE 1

On prépare une lotion démêlante après permanente contenant :

a) un polymère (a) constitué de motifs récurrents de structure

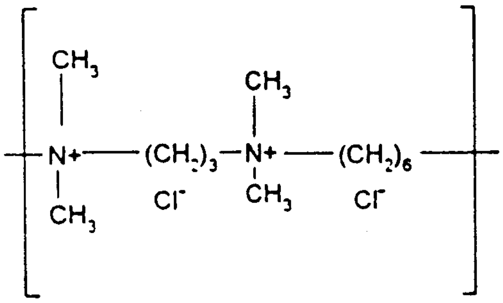

x g

dont le poids moléculaire déterminé par chromatographie par perméation de gel est compris entre 9500 et 9900.

b) un copolymère de chlorure de diallyldiméthylammonium et d'acide acrylique, à 35 % de matière active (MA) vendu sous la dénomination MERQUAT 280 ® par la Société CALGON y g

HCl qs pH 5,5

Conservateurs qs

Eau qsp 100 g

Cinq lotions de ce type : I, II, III, IV, V, présentant 5 rapports en polymères $\frac{(a)}{(b)}$ différents, respectivement :1,5 / 1 / 0,75 / 0,5 / 0,1 ont chacune été comparées à des lotions IA et IB pour la lotion I, IIA et IIB pour la lotion II, IIIA et IIIB pour la lotion III, IVA et IVB pour la lotion IV, VA et VB pour la lotion V, les lotions A contenant uniquement le polymère (a) à la concentration x + y, les lotions B contenant uniquement le polymère (b) à la concentration x + y.

La composition en polymères de ces lotions est exprimée dans le tableau ci-après :

| LOTIONS / POLYMERES | I | IA | IB | II | IIA | IIB | III | IIIA | IIIB | IV | IVA | IVB | V | VA | VB |
|---|---|---|---|---|---|---|---|---|---|---|---|---|---|---|---|
| (x) en g | 1,05 | 1,75 | - | 1 | 2 | - | 0,75 | 1,75 | - | 0,5 | 1,5 | - | 0,1 | 1,1 | - |
| (y) en g | 0,7 | - | 1,75 | 1 | - | 2 | 1 | - | 3,75 | 1 | - | 1,5 | 1 | - | 1,1 |
| Rapport $\frac{(a)}{(b)}$ | 1,5 | - | - | 1 | - | - | 0,75 | - | - | 0,5 | - | - | 0,1 | - | - |

Des mèches de 2,5 g de cheveux permanentés chacune sont traitées respectivement avec les 15 compositions du tableau ci-dessus, puis rincées à l'eau après 2 minutes de pause.

On a ensuite comparé à l'aide d'un test d'évaluation sensorielle, le démêlage à l'état mouillé des cheveux traités par ces lotions.

Le test utilisé a pour objet le classement, par un jury, de chaque série de 3 échantillons en fonction croissante ou

décroissante de l'efficacité du démêlage. Les 3 mèches de la même série sont présentées simultanément au juge. On lui demande de les classer des plus faciles à démêler aux plus difficiles. L'analyse statistique des résultats est effectuée à l'aide des tables de A. KRAMER (Food Technology 17 - (12), 124 - 125 1963).

**RESULTATS :**

| JUGES / LOTIONS | 1 | 2 | 3 | 4 | 5 | 6 | 7 | 8 | 9 | 10 | SOMME DES RANGS |
|---|---|---|---|---|---|---|---|---|---|---|---|
| I | 2 | 2 | 2 | 2 | 2 | 2 | 2 | 2 | 2 | 2 | 20 |
| IA | 3 | 3 | 3 | 3 | 3 | 3 | 3 | 3 | 3 | 3 | 30 |
| IB | 1 | 1 | 1 | 1 | 1 | 1 | 1 | 1 | 1 | 1 | 10 |
| II | 2 | 2 | 2 | 2 | 1 | 2 | 2 | 2 | 2 | 2 | 19 |
| IIA | 3 | 3 | 3 | 3 | 3 | 3 | 3 | 3 | 3 | 3 | 30 |
| IIB | 1 | 1 | 1 | 1 | 2 | 1 | 1 | 1 | 1 | 1 | 11 |
| III | 1 | 1 | 1 | 1 | 1 | 1 | 1 | 1 | 1 | 1 | 10 |
| IIIA | 3 | 3 | 3 | 3 | 3 | 3 | 3 | 3 | 3 | 3 | 30 |
| IIIB | 2 | 2 | 2 | 2 | 2 | 2 | 2 | 2 | 2 | 2 | 20 |
| IV | 1 | 1 | 1 | 2 | 1 | 1 | 1 | 1 | 1 | 1 | 11 |
| IVA | 2 | 2 | 2 | 1 | 2 | 2 | 2 | 2 | 2 | 2 | 19 |
| IVB | 3 | 3 | 3 | 3 | 3 | 3 | 3 | 3 | 3 | 3 | 30 |
| V | 1 | 1 | 1 | 1 | 1 | 2 | 1 | 1 | 1 | 1 | 11 |
| VA | 2 | 2 | 2 | 2 | 2 | 1 | 2 | 2 | 2 | 2 | 19 |
| VB | 3 | 3 | 3 | 3 | 3 | 3 | 3 | 3 | 3 | 3 | 30 |

**CONCLUSION :**

Pour les lotions III à V selon l'invention, les résultats obtenus avec les mèches traitées par les lotions contenant le mélange des polymères (a) et (b) sont significativement supérieurs à ceux obtenus avec les deux autres lotions contenant chacun des polymères utilisé seul, au seuil de 5 %.

Les lotions III, IV et V pour lesquelles le rapport $\frac{(a)}{(b)}$ est respectivement de 0,75 ; 0,5 ; 0,1 présentent un effet de synergie par rapport aux lotions I et II avec des rapports en polymères $\frac{(a)}{(b)}$ de 1,5 et de 1.

EXEMPLE 2

La lotion IV de l'exemple 1 contenant 0,5 g de polymère (a) et 1 g de polymère (b) a été comparée à une lotion IV C ne renfermant que 0,5 g de polymère (a).

Des mèches de cheveux permanentés de 2,5 g sont traitées respectivement avec les lotions IV et IVC, rincées à l'eau après 5 minutes de pose, puis séchées au casque séchoir. On procède à une seconde application des lotions, rinçage et séchage, et à l'aide du test d'évaluation sensorielle décrit à l'exemple 1, on demande au juge de classer les mèches en fonction de l'alourdissement (effet de charge par le polymère).

RESULTATS

| JUGES / LOTIONS | 1 | 2 | 3 | 4 | 5 | 6 | 7 | 8 | 9 | 10 | SOMME DES RANGS |
|---|---|---|---|---|---|---|---|---|---|---|---|
| IV | 1 | 1 | 1 | 1 | 1 | 1 | 1 | 1 | 1 | 1 | 10 |
| IVC | 2 | 2 | 2 | 2 | 2 | 2 | 2 | 2 | 2 | 2 | 20 |

Des résultats strictement identiques sont obtenus lorsqu'on effectue un shampooing entre les deux applications de lotions.

CONCLUSION :

La lotion IV selon l'invention n'alourdit pas les cheveux lors d'applications répétées contrairement à la lotion IVC ne renfermant que le polymère (a) à 0,5 %.

Des résultats identiques sont obtenus comparativement à une lotion IVD ne renfermant que le polymère (a) à 1,5%.

EXEMPLE 3

On prépare un shampooing de composition suivante :

- Alkyl ($C_9$-$C_{10}$-$C_{11}$ / 20-40-40) polyglycoside vendu à 50 % de MA par la société HENKEL sous la dénomination APG 300® 15 gMA

- Polymère de formule (a) constitué de motifs récurrents de structure :

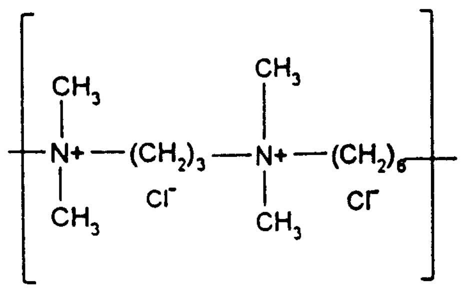

dont le poids moléculaire déterminé par chromatographie par perméation de gel est compris entre 9500 et 9900. 0,2 g

- Copolymère de chlorure de diallyldiméthylammonium et d'acide acrylique, vendu sous la dénomination MERQUAT 280® par la Société CALGON à 35 % de MA 1 g MA
- Acide chlorhydrique qs pH 5
- Conservateurs, parfum, colorant qs
- Eau qsp 100 g

EXEMPLE 4

On prépare un shampooing de composition suivante :

| | |
|---|---|
| - Acide alkyl (C12-C14/70-30) éther carboxylique polyoxyéthyléné à 4,5 moles d'OE, vendu à 90 % de matière active (MA) sous la dénomination AKYPO RLM 45® par la société CHEM'Y | 12 g MA |
| - Cocoamphocarboxyglycinate (CTFA, 3ème édition, 1982) vendu sous la dénomination MIRANOL C2M® Conc., par la Société MIRANOL en solution aqueuse à 38 % de matière active (MA) | 8 g MA |
| - Polymère de formule (a) tel que décrit à l'exemple 3 | 0,75 g |
| - Copolymère de chlorure de diallyldiméthylammonium et d'acide acrylique, vendu sous la dénomination MERQUAT 280® par la Société CALGON à 35 % de MA | 1 g MA |
| - Oléate de propylèneglycol oxyéthylené à 55 moles d'oxyde d'éthylène et estérifié par l'acide oléique, vendu en solution à 43,6 % de matière active sous la dénomination ANTIL 141 LIQUID® par la Société GOLDSCHMIDT | 1,2 g MA |
| - NaOH qs | pH 7 |
| - Conservateurs, parfum, qs | |
| - Eau qsp | 100 g |

EXEMPLE 5

On prépare un shampooing de composition suivante :

| | |
|---|---|
| - Laurylsulfate de triéthanolamine en solution à 40 % MA | 16 g MA |
| - Monoisopropanolamide d'acide de coprah | 2 g |
| - Polymère de formule (a) tel que décrit à l'exemple 3 | 0,5 g |
| - Ether d'alcool gras de formule : $C_{16}H_{33}$-0[$C_2H_3$(OH)]($CH_2$)$_2$-$C_{14}H_{29}$ | 2,5 g |
| - Copolymère de chlorure de diallyldiméthylammonium et d'acide acrylique, vendu sous la dénomination MERQUAT 280® par la Société CALGON à 35 % de MA | 1,6 g MA |
| - Conservateurs, parfum qs | |
| - Acide chlorhydrique qs | pH 4 |
| - Eau qsp | 100 g |

EXEMPLE 6

On prépare un après-shampooing de composition suivante :

| | |
|---|---|
| - Polymère de formule (a) tel que décrit à l'exemple 30, | 75 g |
| - Copolymère de chlorure de diallyldiméthylammonium et d'acide acrylique, vendu sous la dénomination MERQUAT 280® par la Société CALGON à 35 % de MA | 1 g MA |
| - Chlorure de béhényltriméthylammonium vendu sous la dénomination commerciale "GENAMIN KDM-F par la Société HOECHST | 2 g |
| - Mélange (80/20) d'alcool cétylstéarylique et d'alcool cétylstéarylique oxyéthyléné à 33 moles d'oxyde d'éthylène vendu sous la dénomination SINNOWAX AO® par la Société HENKEL | 3 g |
| - Acide chlorhydrique qs | pH 4 |
| - Eau qsp | 100 g |

EXEMPLE 7

On prépare un après-shampooing de composition suivante :

| | |
|---|---|
| - Chlorure de triméthylcétylammonium vendu en solution à 25 % MA par la Société HENKEL sous la dénomination DEHYQUART A® | 3 g MA |
| - Polymère de formule (a) tel que décrit à l'exemple 3 | 1 g |
| - Copolymère de chlorure de diallyldiméthylammonium et d'acide acrylique, vendu sous la dénomination MERQUAT 280® par la Société CALGON à 35 % de MA | 2,5 g MA |
| - Gomme de guar hydroxypropylée vendue par la société MEYHALL sous la dénomination JAGUAR HP8® | 1 g |
| - Polyéthylèneglycol (150 OE) vendu par la Société UNION CARBIDE sous la dénomination CARBOWAX 8000® | 2,5 g |
| - Acide chlorhydrique qs | pH 4 |
| - Conservateur, parfum, qs | |
| - Eau qsp | 100 g |

EXEMPLE 8

On prépare un gel pour la douche de composition suivante :

| | |
|---|---|
| - Acide alkyl (C12-C14/70-30) éther carboxylique polyoxyéthyléné à 4,5 moles d'OE, vendu à 90 % de matière active (MA) sous la dénomination AKYPO RLM 45® par la société CHEM'Y | 15 g MA |
| - Lauryléthersulfate de sodium oxyéthyléné à 2,2 moles d'OE, vendu en solution à 28 % MA | 10 g MA |
| - Polymère de formule (a) tel que décrit à l'exemple 3 | 0,05 g |
| - Copolymère de chlorure de diallyldiméthylammonium et d'acide acrylique, vendu sous la dénomination MERQUAT 280® par la Société CALGON à 35 % de MA | 0,1 g MA |
| - Glycérine | 2g |
| - NaOH qs | pH 7 |
| - Conservateurs, parfum, qs | |
| - Eau qsp | 100 g |

EXEMPLE 9

On prépare un gel douche de composition suivante :

- Lauryl($C_{12}$-$C_{14}$/70-30)éthersulfate de sodium oxyéthyléné à 2,2 moles d'oxyde d'éthylène vendu à 28 % de matière active (MA) — 20 g MA
- Cocamidopropylbétaïne vendue à 30 % de MA par la Société GOLDSCHMIDT sous la dénomination TEGOBETAIN HS® — 5 g MA
- Polymère de formule (a) constitué de motifs récurrents de structure :

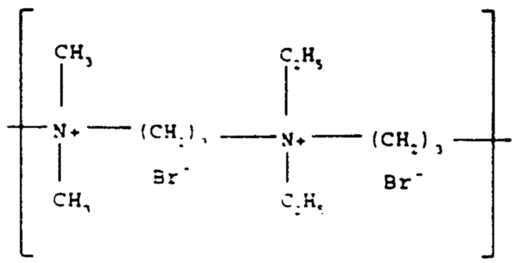

dont le poids moléculaire déterminé par chromatographie par perméation de gel est d'environ 1200 — 0,5 g MA

- Copolymère de chlorure de diallyldiméthylammonium et d'acide acrylique, vendu sous la dénomination MERQUAT 280® par la Société CALGON à 35 % de MA — 1 g MA
- NaOH qs — pH 7
- Conservateur : qs
- Eau qsp — 100 g

## Revendications

1. Composition cosmétique comprenant:

   - au moins un polymère (a) de polyammonium quaternaire constitué de motifs récurrents de formule :

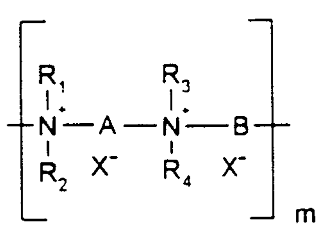

(a)

dans laquelle :

- A et B, identiques ou différents, peuvent représenter des groupements polyméthyléniques contenant de 2 à 20 atomes de carbone pouvant être linéaires ou ramifiés, saturés ou insaturés et pouvant contenir, intercalés dans la chaîne principale un ou plusieurs groupements $-CH_2-Y-CH_2-$ avec Y désignant 0, S, SO, $SO_2$ ou -

CHOH-

- $X^-$ est un anion dérivé d'un acide minéral ou organique.
- m est tel que la masse moléculaire est comprise entre 1 000 et 100 000
- $R_1$, $R_2$, $R_3$ et $R_4$, identiques ou différents désignent un radical alkyle ou hydroxyalkyle ayant de 1 à 4 atomes de carbone environ.
- et au moins un polymère (b) constitué de 70 % à 90 % en poids d'unités diallyldialkylammonium dans lesquelles le radical alkyle contient de 1 à 18 atomes de carbone et de 30 % à 10 % en poids d'unités acrylique ou méthacrylique, le rapport en poids entre le polymère a) et le copolymère b) étant strictement inférieur à 1.

**2.** Composition selon la revendication 1, caractérisée par le fait que le polymère (a) est constitué de motifs récurrents répondant à la formule :

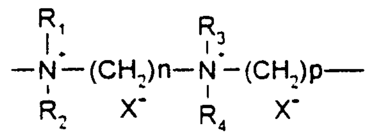

(a)

dans laquelle $R_1$, $R_2$, $R_3$, $R_4$, identiques ou différents désignent un radical alkyle ayant de 1 à 4 atomes de carbone, n et p sont des nombres entiers compris entre 2 et 20, $X^-$ est un anion dérivé d'un acide minéral ou organique.

**3.** Composition selon l'une quelconque des revendications précédentes, caractérisée par le fait que les radicaux $R_1$, $R_2$, $R_3$, $R_4$ représentent un radical méthyle ou éthyle.

**4.** Composition selon l'une quelconque des revendications précédentes, caractérisée par le fait que X est choisi parmi le groupe comprenant le chlore, l'iode et le brome.

**5.** Composition selon l'une quelconque des revendications 1 à 4, caractérisée par le fait que le polymère (a) est tel que $R_1$, $R_2$, $R_3$ et $R_4$ représentent le radical méthyle, n = 3, p = 6 et X représente un atome de chlore.

**6.** Composition selon l'une quelconque des revendications 1 à 4, caractérisée par le fait que le polymère (a) est tel que $R_1$ et $R_2$ représentent un radical méthyle, $R_3$ et $R_4$ représentent un radical éthyle, n = p = 3 et X représente un atome de brome.

**7.** Composition selon l'une quelconque des revendications précédentes, caractérisée par le fait que le poids moléculaire du copolymère b), déterminé par chromatographie par perméation de gel est compris entre 50.000 et 10.000.000 et de préférence entre 200.000 et 5.000.000.

**8.** Composition selon l'une quelconque des revendications 1 à 7, caractérisée par le fait que le copolymère b) est un copolymère de chlorure de diallyldiméthylammonium ou de diallyldiéthyl-ammonium et d'acide acrylique d'un poids moléculaire compris entre 200.000 et 5.000.000.

**9.** Composition selon l'une quelconque des revendications 1 à 8, caractérisée par le fait que le rapport pondéral entre le polymère a) et le copolymère b) est inférieur ou égal à 0,75 et de préférence inférieur ou égal à 0,5.

**10.** Composition selon l'une quelconque des revendications 1 à 9, caractérisée par le fait qu'elle contient le polymère a) en des proportions variant entre 0,05 % et 4 % en poids et de préférence entre 0,1 % et 3 % en poids.

**11.** Composition selon l'une quelconque des revendications 1 à 10 caractérisée par le fait qu'elle contient le copolymère b) en des proportions variant entre 0,1 % et 8 % en poids et de préférence entre 0,2 % et 6 % en poids.

**12.** Composition selon l'une quelconque des revendications 1 à 11, caractérisée par le fait qu'elle présente un pH compris entre 4 et 8, et de préférence entre 5 et 7.

**13.** Composition selon l'une quelconque des revendications 1 à 12, caractérisée par le fait qu'elle contient au moins un agent tensio-actif anionique, cationique, non ionique, amphotère, zwitterionique ou leurs mélanges.

**14.** Composition selon l'une quelconque des revendications 1 à 13, caractérisée par le fait que le ou les agent(s) tensio-actif(s) est (sont) présent(s) à une concentration comprise entre 0,1 % et 40 % en poids par rapport au poids total de la composition.

**15.** Composition selon l'une quelconque des revendications 1 à 14, caractérisée par le fait qu'elle contient au moins un additif choisi parmi les parfums, les solvants, les agents conservateurs, les agents séquestrants, les agents épaississants, les agents adoucissants, les agents modificateurs de mousse, les agents acidifiants ou alcalinisants, les colorants, les agents modificateurs de viscosité, les agents nacrants, les agents hydratants, les agents anti-pelliculaires, les agents anti-séborrhéiques, les filtres solaires, les silicones volatiles ou non, organomodifiées ou non, les agents de conditionnements différents des polymères a) et b) tels que des composés cationiques polymériques ou non polymériques ou les huiles hydrocarbonées, les protéines.

**16.** Composition selon l'une quelconque des revendications 1 à 15, caractérisée par le fait qu'elle se présente sous forme de shampooing, d'après-shampooing à rincer, de composition pour la permanente, le défrisage, la coloration ou la décoloration des cheveux, de composition à rincer à appliquer entre les deux étapes d'une permanente ou d'un défrisage, de composition lavante pour le corps, de lotions.

**17.** Utilisation cosmétique d'une composition telle que définie dans l'une quelconque des revendications 1 à 16, pour le soin des cheveux et/ou de la peau.

## Claims

**1.** Cosmetic composition containing:

- at least one quaternary polyammonium polymer (a) consisting of recurring units of formula:

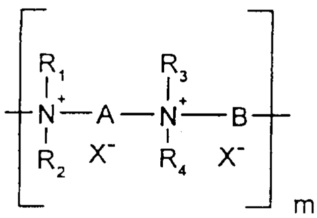

(a)

in which:

- A and B, which may be identical or different, can represent polymethylene groups containing 2 to 20 carbon atoms which may be linear or branched, saturated or unsaturated and which may contain, intercalated in the principal chain, one or more $-CH_2-Y-CH_2-$ groups with Y designating O, S, SO, $SO_2$ or -CHOH-,
- $X^-$ is an anion derived from an inorganic or organic acid,
- m is such that the molecular mass is between 1000 and 100,000,
- $R_1$, $R_2$, $R_3$ and $R_4$, which are identical or different, designate an alkyl or hydroxyalkyl radical having about 1 to 4 carbon atoms,
  and
- at least one polymer (b) consisting of 70 to 90 % by weight of diallyldialkylammonium units in which the alkyl radical contains 1 to 18 carbon atoms, and 30 % to 10 % by weight of acrylic or methacrylic units, the weight ratio between the (a) polymer and the (b) copolymer being strictly less than 1.

**2.** Composition according to Claim 1, characterized by the fact that the (a) polymer consists of recurring units corresponding to the formula:

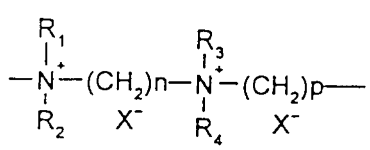

(a)

in which $R_1$, $R_2$, $R_3$ and $R_4$, which are identical or different, designate an alkyl radical having 1 to 4 carbon atoms, n and p are integers ranging from 2 to 20, and $X^-$is an anion derived from an inorganic or organic acid.

3. Composition according to either of the preceding claims, characterized by the fact that the $R_1$, $R_2$, $R_3$ and $R_4$ radicals represent a methyl or ethyl radical.

4. Composition according to any one of the preceding claims, characterized by the fact that X is chosen from the group comprising chlorine, iodine and bromine.

5. Composition according to any one of Claims 1 to 4, characterized by the fact that the (a) polymer is such that $R_1$, $R_2$, $R_3$ and $R_4$ represent the methyl radical, n = 3, p = 6 and X represents a chlorine atom,

6. Composition according to any one of Claims 1 to 4, characterized by the fact that the (a) polymer is such that $R_1$, $R_2$ represent a methyl radical, $R_3$ and $R_4$ represent an ethyl radical, n = p = 3 and X represents a bromine atom.

7. Composition according to any one of the preceding claims, characterized by the fact that the molecular weight of the b) copolymer determined by gel permeation chromatography, is between 50,000 and 10,000,000 and preferably between 200,000 and 5,000,000.

8. Composition according to any one of Claims 1 or 7, characterized by the fact that the b) copolymer is a copolymer of diallyldimethylammonium or diallydiethylammonium chloride and acrylic acid having a molecular weight of between 200,000 and 5,000,000.

9. Composition according to any one of Claims 1 to 8, characterized by the fact that the weight ratio between the a) polymer and the b) copolymer is less than or equal to 0.75 and preferably less than or equal to 0.5.

10. Composition according to any one of Claims 1 to 9, characterized by the fact that it contains the a) polymer in proportions varying between 0.05 % and 4 % by weight and preferably between 0.1 % and 3 % by weight.

11. Composition according to any one of Claims 1 to 10, characterized by the fact that it contains the b) copolymer in proportions varying between 0.1 % and 8 % by weight and preferably between 0.2 % and 6 % by weight.

12. Composition according to any one of Claims 1 to 11, characterized by the fact that it has a pH of between 4 and 8, and preferably between 5 and 7.

13. Composition according to any one of Claims 1 to 12, characterized by the fact that it contains at least one anionic, cationic, non-ionic, amphoteric or zwitterionic surfactant or mixtures thereof.

14. Composition according to any one of Claims 1 to 13, characterized by the fact that the surfactant(s) is (are) present at a concentration of between 0.1 % and 40 % by weight relative to the total weight of the composition.

15. Composition according to any one of Claims 1 to 14, characterized by the fact that it contains at least one additive chosen from perfumes, solvents, preservatives, sequestrants, thickeners, emollients, foam-modifying agents, acidifying or alkalinizing agents, dyes, viscosity-modifying agents, pearlescent agents, moisturizing agents, anti-dandruff agents, anti-seborrhoeic agents, sunscreens, volatile or non-volatile silicones, organomodified or otherwise, conditioning agents different from the a) and b) polymers such as polymeric or non-polymeric cationic compounds or hydrocarbon oils and proteins.

16. Composition according to any one of Claims 1 to 15, characterized by the fact that it is in the form of a shampoo, after-shampoo to be rinsed off, composition for permanent waving and straightening, dyeing or bleaching of the hair, rinse-off composition to be applied between the two stages of a permanent waving or of a hair straightening, washing composition for the body, lotions.

17. Cosmetic use of a composition as defined in any one of Claims 1 to 16, for hair and/or skin care.

**Patentansprüche**

1. Kosmetische Zusammensetzung, umfassend

- mindestens ein Polymeres (a) von quaternärem Polyammonium aus Struktureinheiten der Formel:

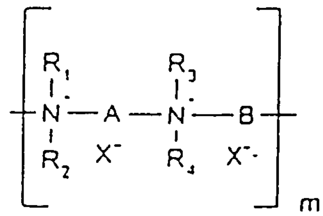

(a)

in der

- A und B gleich oder verschieden sind und Polymethylengruppen mit 2 bis 20 Kohlenstoffatomen bedeuten können, die linear oder verzweigt und gesättigt oder ungesättigt sein können und in deren Hauptkette eine oder mehrere Gruppen der Formel $-CH_2-Y-CH_2$, in der Y die Bedeutung O, S, SO, $SO_2$ oder -CHOH- hat, eingeschoben sein können,
- $X^-$ ein von einer anorganischen oder organischen Säure abgeleitetes Anion bedeutet,
- m einen solchen Wert hat, daß die Molekülmasse einen Wert von 1000 bis 100 000 aufweist,
- $R_1$, $R_2$, $R_3$ und $R_4$, die gleich oder verschieden sind, einen Alkyl- oder Hydroxyalkylrest mit 1 bis etwa 4 Kohlenstoffatomen bedeuten,
- und mindestens ein Polymeres (b), das aus 70 bis 90 Gew.-% Diallyldialkylammonium-Einheiten, bei denen der Alkylrest 1 bis 18 Kohlenstoffatome aufweist, und aus 30 bis 10 Gew.-% Acryl- oder Methacryleinheiten besteht, wobei das Gewichtsverhältnis des Polymeren (a) zum Copolymeren (b) deutlich unter 1 liegt.

2. Zusammensetzung nach Anspruch 1, dadurch gekennzeichnet, daß das Polymere (a) aus Struktureinheiten der folgenden Formel besteht:

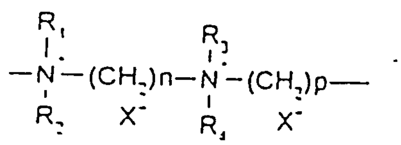

(a)

in der $R_1$, $R_2$, $R_3$ und $R_4$ gleich oder verschieden sind und einen Alkylrest mit 1 bis 4 Kohlenstoffatomen bedeuten, n und p ganze Zahlen von 2 bis 20 bedeuten und $X^-$ ein Anion bedeutet, das sich von einer anorganischen oder organischen Säure ableitet.

3. Zusammensetzung nach einem der vorstehenden Ansprüche, dadurch gekennzeichnet, daß die Reste $R_1$, $R_2$, $R_3$ und $R_4$ einen Methyl- oder Ethylrest bedeuten.

4. Zusammensetzung nach einem der vorstehenden Ansprüche, dadurch gekennzeichnet, daß X aus der Gruppe Chlor, Iod und Brom ausgewählt ist.

5. Zusammensetzung nach einem der Ansprüche 1 bis 4, dadurch gekennzeichnet, daß das Polymere (a) so beschaffen ist, daß $R_1$, $R_2$, $R_3$ und $R_4$ einen Methylrest bedeuten, n den Wert 3 hat, p den Wert 6 hat und X ein Chloratom bedeutet.

6. Zusammensetzung nach einem der Ansprüche 1 bis 4, dadurch gekennzeichnet, daß das Polymere (a) so beschaffen ist, daß $R_1$ und $R_2$ einen Methylrest bedeuten, $R_3$ und $R_4$ einen Ethylrest bedeuten, n und p jeweils den Wert 3 haben und X ein Bromatom bedeutet.

7. Zusammensetzung nach einem der vorstehenden Ansprüche, dadurch gekennzeichnet, daß das Molekulargewicht des Copolymeren (b), bestimmt durch Gelpermeationschromatographie 50 000 bis 10 000 000 und vorzugsweise 200 000 bis 5 000 000 beträgt.

8. Zusammensetzung nach einem der Ansprüche 1 bis 7, dadurch gekennzeichnet, daß es sich beim Copolymeren

(b) um ein Copolymeres aus Diallylmethylammoniumchlorid oder Diallyldiethylammoniumchlorid und Acrylsäure mit einem Molekulargewicht von 200 000 bis 5 000 000 handelt.

**9.** Zusammensetzung nach einem der Ansprüche 1 bis 8, dadurch gekennzeichnet, daß das Gewichtsverhältnis des Polymeren (a) zum Polymeren (b) 0,75 oder weniger und vorzugsweise 0,5 oder weniger beträgt.

**10.** Zusammensetzung nach einem der Ansprüche 1 bis 9, dadurch gekennzeichnet, daß sie das Polymere (a) in einem Anteil von 0,05 bis 4 Gew.-% und vorzugsweise von 0,1 bis 3 Gew.-% enthält.

**11.** Zusammensetzung nach einem der Ansprüche 1 bis 10, dadurch gekennzeichnet, daß sie das Copolymere (b) in einem Anteil von 0,1 bis 8 Gew.-% und vorzugsweise von 0,2 bis 6 Gew.-% enthält.

**12.** Zusammensetzung nach einem der Ansprüche 1 bis 11, dadurch gekennzeichnet, daß sie einen pH-Wert von 4 bis 8 und vorzugsweise von 5 bis 7 aufweist.

**13.** Zusammensetzung nach einem der Ansprüche 1 bis 12, dadurch gekennzeichnet, daß sie mindestens ein anionisches, kationisches, nicht-ionisches, amphoteres oder zwitterionisches oberflächenaktives Mittel oder ein Gemisch davon enthält.

**14.** Zusammensetzung nach einem der Ansprüche 1 bis 13, dadurch gekennzeichnet, daß das oder die oberflächenaktiven Mittel in einer Konzentration von 0,1 bis 40 Gew.-%, bezogen auf das Gesamtgewicht der Zusammensetzung, vorhanden sind.

**15.** Zusammensetzung nach einem der Ansprüche 1 bis 14, dadurch gekennzeichnet, daß sie mindestens ein Additiv enthält, das unter Parfums, Lösungsmitteln, Konservierungsmitteln, Maskierungsmitteln, Verdickungsmitteln, reizlindernden Mitteln, Schaummodifikationsmitteln, ansäuernden oder alkalisierenden Mitteln, farbgebenden Mitteln, Viskositätsmodifikationsmitteln, Glanzmitteln, hydratisierenden Mitteln, Mitteln gegen Schuppen, Mitteln gegen Seborrhoe, Sonnenschutzfiltern, flüchtigen oder nicht-flüchtigen, organisch modifizierten oder nicht-modifizierten Siliconen, von den Polymeren (a) und (b) unterschiedlichen Konditionierungsmitteln, wie kationischen, polymeren oder nicht-polymeren Verbindungen, Kohlenwasserstoffölen und Proteinen ausgewählt ist.

**16.** Zusammensetzung nach einem der Ansprüche 1 bis 15, dadurch gekennzeichnet, daß sie in Form eines Shampoos, eines nach der Shampoobehandlung einzusetzenden Spülmittels, einer Zusammensetzung zur Dauerwellenbehandlung, Entkrausung, Färbung oder Entfärbung von Haaren, einer Spülzusammensetzung zur Anwendung zwischen den beiden Stufen einer Dauerwellen- oder Entkrausungsbehandlung, einer Waschzusammensetzung für den Körper oder einer Lotion vorliegt.

**17.** Kosmetische Verwendung einer Zusammensetzung gemäß einem der Ansprüche 1 bis 16 zur Haarpflege und/oder Hautpflege.